# EUROPEAN PATENT APPLICATION

(11) **EP 2 241 563 A1**
(43) Date of publication of application: **20.10.2010**
(21) Application number: 09704815.1
(22) Date of filing: 16.01.2009
(51) Int. Cl.: C07D 333/18, H01L 51/05, H01L 51/30

(54) **TRI-SUBSTITUTED AROMATIC COMPOUND**

(30) Priority: 25.01.2008 JP 2008014915
(71) Applicant: Keio University, Tokyo 108-8345 (JP); Sumitomo Chemical Company, Limited, Tokyo 104-8260 (JP)
(72) Inventor: KAKIUCHI, Fumitoshi, Yokohama-shi Kanagawa 223-8522 (JP); OGUMA, Jun, Tsukuba-shi Ibaraki 305-0051 (JP)
(74) Representative: Duckworth, Timothy John
(86) International application number: PCT/JP2009/050531
(87) International publication number: WO 2009/093526

(57) **Abstract**

A compound represented by the following formula (1): 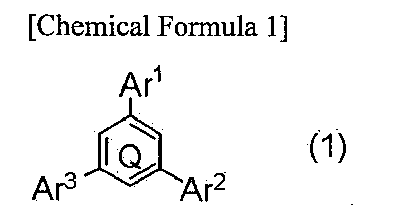 wherein Ar¹, Ar² and Ar³ each independently represent an aryl group or a monovalent heterocyclic group; the aryl group or the monovalent heterocyclic group represented by Ar¹ has an aryl group, a monovalent alicyclic hydrocarbon group or a monovalent heterocyclic group at one position adjacent to the atom that forms Ar¹ and is bonded to a carbon atom forming the benzene ring Q; the aryl group or the monovalent heterocyclic group represented by Ar² has an aryl group, a monovalent alicyclic hydrocarbon group or a monovalent heterocyclic group at one position adjacent to the atom that forms Ar² and is bonded to a carbon atom forming the benzene ring Q; the aryl group or the monovalent heterocyclic group represented by Ar³ has an aryl group, a monovalent alicyclic hydrocarbon group or a monovalent heterocyclic group at one position adjacent to the atom that forms Ar³ and is bonded to a carbon atom forming the benzene ring Q.

## Description

### Technical Field

The present invention relates to a tri-substituted aromatic compound and a method for producing it.

### Background Art

Aromatic compounds exhibit a charge transport property by the use of conjugate planes, and they are therefore useful as materials for production of organic ELs, organic transistors and the like. Of these, compounds having a concentrically extending structure around a single atom or a single molecule as the center are particularly useful as organic semiconductor layers for organic transistors, because they exhibit self-aggregation and orientation properties, and charge transport properties in the conjugate planes. Such compounds have been proposed, such as those represented by the following formula, for example (Patent document 1).

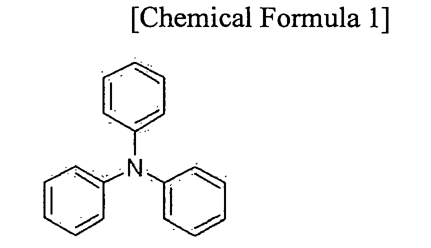

[Patent document 1] Japanese Unexamined Patent Application Publication No. 2007-256753

### Disclosure of the Invention

### Problems to be Solved by the Invention

The aforementioned compounds, however, do not have sufficient charge injection and transport properties.

It is an object of the present invention to provide compounds with excellent charge injection and transport properties, as well as a method for producing them.

### Means for Solving the Problems

The invention provides, firstly, a compound represented by the following formula (1).

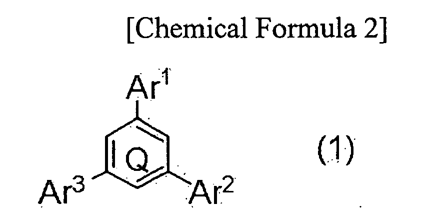

In the formula, Ar¹, Ar² and Ar³ each independently represent an aryl group or a monovalent heterocyclic group. The aryl group or the monovalent heterocyclic group represented by Ar¹ has an aryl group, a monovalent alicyclic hydrocarbon group or a monovalent heterocyclic group at one position adjacent to the atom that forms Ar¹ and is bonded to a carbon atom forming the benzene ring Q. The aryl group or the monovalent heterocyclic group represented by Ar² has an aryl group, a monovalent alicyclic hydrocarbon group or a monovalent heterocyclic group at one position adjacent to the atom that forms Ar² and is bonded to a carbon atom forming the benzene ring Q. The aryl group or the monovalent heterocyclic group represented by Ar³ has an aryl group, a monovalent alicyclic hydrocarbon group or a monovalent heterocyclic group at one position adjacent to the atom that forms Ar³ and is bonded to a carbon atom forming the benzene ring Q.

The invention provides, secondly, a method for producing a compound represented by any of the following formulas (3-1) to (3-3), which comprises making the compound undergo a cyclization reaction in the presence of an acid or a base.

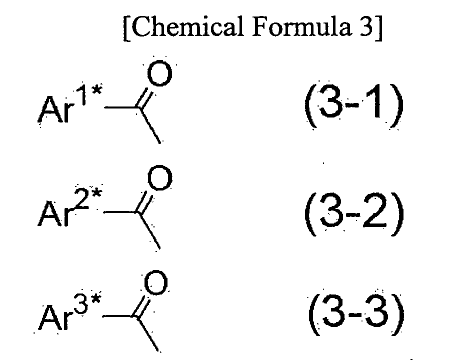

In the formulas, Ar^{1*}, Ar^{2*} and Ar^{3*} each independently represent an aryl group or a monovalent heterocyclic group. The aryl group or the monovalent heterocyclic group represented by Ar^{1*} has an aryl group, a monovalent alicyclic hydrocarbon group or a monovalent heterocyclic group at one position adjacent to the atom that forms Ar^{1*} and is bonded to the acetyl group. The aryl group or the monovalent heterocyclic group represented by Ar^{2*} has an aryl group, a monovalent alicyclic hydrocarbon group or a monovalent heterocyclic group at one position adjacent to the atom that forms Ar^{2*} and is bonded to the acetyl group. The aryl group or the monovalent heterocyclic group represented by Ar^{3*} has an aryl group, a monovalent alicyclic hydrocarbon group or a monovalent heterocyclic group at one position adjacent to the atom that forms Ar^{3*} and is bonded to the acetyl group.

### Effect of the Invention

The compounds of the invention have excellent charge injection and transport properties. In addition, the compounds of the invention generally have excellent stability in air, light stability, acid resistance and heat resistance. According to the production method of the invention it is possible to easily produce the compounds described above.

### Best Modes for Carrying Out the Invention

The invention will now be explained in detail.

### <Compounds>

In the above formula (1), the aryl groups represented by Ar¹, Ar² and Ar³ have a carbon number of usually 6-60 and preferably 6-20. Examples of aryl groups include a phenyl group, a naphthalenyl group, an anthracenyl group, a biphenyl group, a fluorenyl group, a triphenyl group, a stilbene-yl group, a distilbene-yl group, a phenanthrene-yl group, a pyrene-yl group and a perylene-yl group.

In the above formula (1), the monovalent heterocyclic groups represented by Ar¹, Ar² and Ar³ have a carbon number of usually 3-60 and preferably 3-20. Examples of monovalent heterocyclic groups include a thienyl group, a thiophenesulfone-yl group, a thiophene sulfoxide-yl group, a pyrrolyl group, a furyl group, a pyridyl group, a thiazolyl group, an oxazolyl group, a thiadiazolyl group, a diazaphenylene-yl group, a quinoline-yl group, a quinoxaline-yl group and a phenanthroline-yl group.

In the above formula (1), Ar¹, Ar² and Ar³ are preferably all monovalent heterocyclic groups from the viewpoint of the electron properties of the molecule, and in consideration of planarity of the molecule, all of the heterocyclic rings composing the monovalent heterocyclic groups are more preferably 5-membered rings, with the heterocyclic rings being most preferably, and each independently, a thiophene ring, a furan ring, a pyrrole ring, a thiazole ring or an oxazole ring.

The aryl groups and the monovalent heterocyclic groups which the aryl groups and the monovalent heterocyclic groups represented by Ar¹, Ar² and Ar³ have on one of the positions adjacent to the atoms that form Ar¹, Ar² and Ar³ and are bonded to carbon atoms of the benzene ring Q, are the same as those mentioned in the explanation of the aryl group above.

The monovalent alicyclic hydrocarbon groups which the aryl groups or the monovalent heterocyclic groups represented by Ar¹, Ar² and Ar³ have on one position adjacent to the atoms that form Ar¹, Ar², Ar³ and are bonded to carbon atoms forming the benzene ring Q, have a carbon number of usually 3-60 and preferably 6-20. Examples of such monovalent alicyclic hydrocarbon groups include a cyclopropane-yl group, a cyclobutane-yl group, a cyclopentane-yl group, a cyclohexane-yl group, a cycloheptane-yl group, a cyclooctane-yl group, a cyclononane-yl group and a cyclodecane-yl group.

The atoms of the aryl group or the monovalent heterocyclic group represented by Ar¹, Ar² and Ar³, other than the aforementioned adjacent positions, optionally have substituents such as an alkyl group, an alkoxy group, an alkylthio group, an alkylamino group, an aryl group, an aryloxy group, an arylalkyl group, an arylalkoxy group, an arylamino group, a monovalent heterocyclic group or a cyano group, or a fluorine atom.

The alkyl group may be straight-chain, branched or cyclic, and the number of carbon atoms will generally be about 1-20. Examples of the alkyl group include a methyl group, an ethyl group, a propyl group, an i-propyl group, a butyl group, an i-butyl group, a t-butyl group, a s-butyl group, a pentyl group, a hexyl group, a cyclohexyl group, a heptyl group, an octyl group, a 2-ethylhexyl group, a nonyl group, a decyl group, a 3,7-dimethyloctyl group and a lauryl group, with a methyl group, a pentyl group, a hexyl group, an octyl group, a 2-ethylhexyl group, a decyl group and a 3,7-dimethyloctyl group being preferred.

The alkoxy group may be straight-chain, branched or cyclic, and the number of carbon atoms will generally be about 1-20. Examples of the alkoxy group include a methoxy group, an ethoxy group, a propyloxy group, an i-propyloxy group, a butoxy group, an i-butoxy group, a t-butoxy group, a s-butoxy group, a pentyloxy group, a hexyloxy group, a cyclohexyloxy group, a heptyloxy group, an octyloxy group, a 2-ethylhexyloxy groupp, a nonyloxy group, a decyloxy group, a 3,7-dimethyloctyloxy group and a lauryloxy group, with a pentyloxy group, a hexyloxy group, an octyloxy group, a 2-ethylhexyloxy group, a decyloxy group and a 3,7-dimethyloctyloxy group being preferred.

The alkylthio group may be straight-chain, branched or cyclic, and the number of carbon atoms will generally be about 1-20. Examples of the alkylthio group include a methylthio group, an ethylthio group, a propylthio group, an i-propylthio group, a butylthio group, an i-butylthio group, a t-butylthio group, a s-butylthio group, a pentylthio group, a hexylthio group, a cyclohexylthio group, a heptylthio group, an octylthio group, a 2-ethylhexylthio group, a nonylthio group, a decylthio group, a 3,7-dimethyloctylthio group and a laurylthio group, with a pentylthio group, a hexylthio group, an octylthio group, a 2-ethylhexylthio group, a decylthio group and a 3,7-dimethyloctylthio group being preferred.

The alkylamino group may be straight-chain, branched or cyclic, and either a monoalkylamino group or dialkylamino group, and the number of carbon atoms will generally be about 1-40. Examples of the alkylamino group include a methylamino group, a dimethylamino group, an ethylamino group, a diethylamino group, a propylamino group, an i-propylamino group, a butylamino group, an i-butylamino group, a t-butylamino group, a s-butylamino group, a pentylamino group, a hexylamino group, a cyclohexylamino group, a heptylamino group, an octylamino group, a 2-ethylhexylamino group, a nonylamino group, a decylamino group, a 3,7-dimethyloctylamino group and a laurylamino group, with a dimethylamino group, a dihexylamino group and a dioctylamino group being preferred.

The aryloxy group will generally have about 6-60 carbon atoms. Examples of the aryloxy group include a phenoxy group, a C₁-C₁₂ alkoxyphenoxy group (where "C₁-C₁₂ alkoxy" means the C1-12 alkoxy portion, same hereunder), a C₁-C₁₂ alkylphenoxy group (where "C₁-C₁₂ alkyl" means the C1-12 alkyl portion, same hereunder), a 1-naphthyloxy group and a 2-naphthyloxy group, with a C₁-C₁₂ alkoxyphenoxy group and a C₁-C₁₂ alkylphenoxy group being preferred.

The arylalkyl group will generally have about 7-60 carbon atoms. Examples of the arylalkyl group include a phenyl-C₁-C₁₂ alkyl group, a C₁-C₁₂ alkoxyphenyl-C₁-C₁₂ alkyl group, a C₁-C₁₂ alkylphenyl-C₁-C₁₂ alkyl group, a 1-naphthyl-C₁-C₁₂ alkyl group and a 2-naphthyl-C₁-C₁₂ alkyl group, with a C₁-C₁₂ alkoxyphenyl-C₁-C₁₂ alkyl group and a C₁-C₁₂ alkylphenyl-C₁-C₁₂ alkyl group being preferred.

The arylalkoxy group will generally have about 7-60 carbon atoms. Examples of the arylalkoxy group include a phenyl-C₁-C₁₂ alkoxy group, a C₁-C₁₂ alkoxyphenyl-C₁-C₁₂ alkoxy group, a C₁-C₁₂ alkylphenyl-C₁-C₁₂ alkoxy group, a 1-naphthyl-C₁-C₁₂ alkoxy group and a 2-naphthyl-C₁-C₁₂ alkoxy group, with a C₁-C₁₂ alkoxyphenyl-C₁-C₁₂ alkoxy group and a C₁-C₁₂ alkylphenyl-C₁-C₁₂ alkoxy group being preferred.

The arylamino group will generally have about 6-60 carbon atoms. Examples of the arylamino group include a phenylamino group, a diphenylamino group, a C₁-C₁₂ alkoxyphenylamino group, a di(C₁-C₁₂ alkoxyphenyl)amino group, a di(C₁-C₁₂ alkylphenyl)amino group, a 1-naphthylamino group and a 2-naphthylamino group, with a C₁-C₁₂ alkylphenylamino group and a di(C₁-C₁₂ alkylphenyl)amino group being preferred.

These aryl groups and these monovalent heterocyclic groups have the same meaning as above.

In the aryl groups or the monovalent heterocyclic groups represented by Ar¹, Ar² and Ar³, the atoms other than the adjacent positions preferably have a hydrogen atom, an alkyl group, an alkoxy group or an alkylthio group, from the viewpoint of solubility in the solvent, and they preferably have an alkyl group, an alkoxy group or an alkylthio group from the viewpoint of molecular heat resistance. From considerations of self-orientation and cohesion of the molecule, these substituents are preferably all identical.

The compounds represented by the above formula (1) are preferably represented by any of the following formulas (2-1) to (2-4), and from the viewpoint of easier synthesis, compounds represented by the formula (2-1) or (2-2) are preferred.

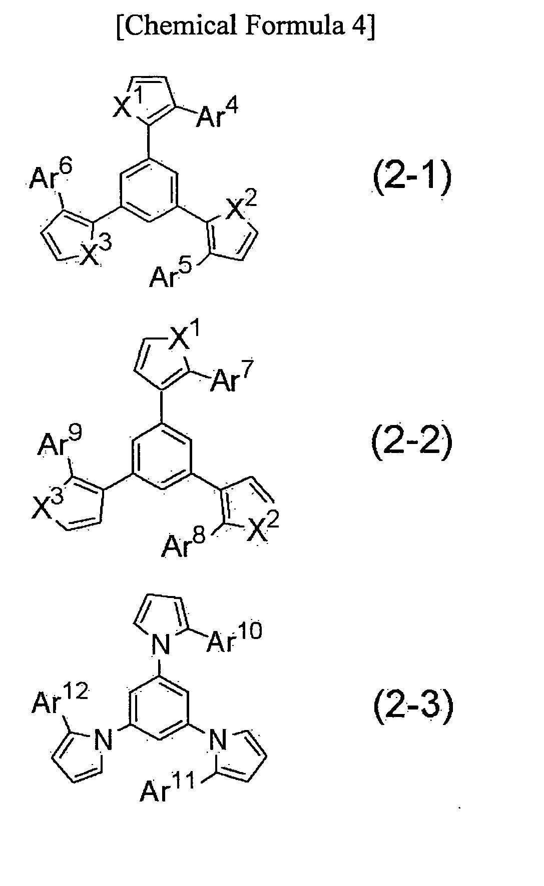

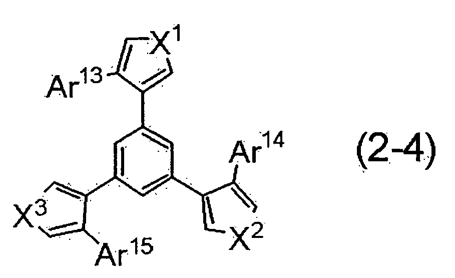

In the formulas, X¹, X² and X³ each independently represent -S(=O)-, -S(=O)₂-, -O- or -N(R²)-. R² represents a hydrogen atom, an alkyl group, an alkoxy group, an alkylthio group, an alkylamino group, an aryl group, an aryloxy group, an arylalkyl group, an arylalkoxy group, an arylamino group, or a monovalent heterocyclic group. Ar⁴, Ar⁵, Ar⁶, Ar⁷, Ar⁸, Ar⁹, Ar¹⁰, Ar¹¹, Ar¹², Ar¹³, Ar¹⁴ and Ar¹⁵ each independently represent an aryl group or a monovalent heterocyclic group.

The aryl groups or the monovalent heterocyclic groups represented by Ar⁴, Ar⁵, Ar⁶, Ar⁷, Ar⁸, Ar⁹, Ar¹⁰, Ar¹¹, Ar¹², Ar¹³, Ar¹⁴ and Ar¹⁵ optionally have substituents such as an alkyl group, an alkoxy group, an alkylthio group, an alkylamino group, an aryl group, an aryloxy group, an arylalkyl group, an arylalkoxy group, an arylamino group, a monovalent heterocyclic group or a cyano group, or a fluorine atom. The atoms in these groups are the same as explained for the substituents mentioned above.

From the viewpoint of molecular planarity, the aryl groups and the monovalent heterocyclic groups represented by Ar⁴, Ar⁵, Ar⁶, Ar⁷, Ar⁸, Ar⁹, Ar¹⁰, Ar¹¹, Ar¹², Ar¹³, Ar¹⁴ and Ar¹⁵ preferably have no alkyl group, alkoxy group, alkylthio group or alkylamino group at the position adjacent to the atom bonded to the atom of the heterocyclic 5-membered ring, such as a pyrrole ring, among the atoms that form the aryl group or the monovalent heterocyclic group.

Ar⁴, Ar⁵, Ar⁶, Ar⁷, Ar⁸, Ar⁹, Ar¹⁰, Ar¹¹, Ar¹², Ar¹³, Ar¹⁴ and Ar¹⁵ are preferably all identical from the viewpoint of molecular symmetry and self-orientation.

From the viewpoint of charge injection and transport properties, X¹, X² and X³ are preferably -S-, -S(=O)- or -S(=O)₂-. They are most preferably -S-.

The following are examples of compounds represented by the above formula (2-1).

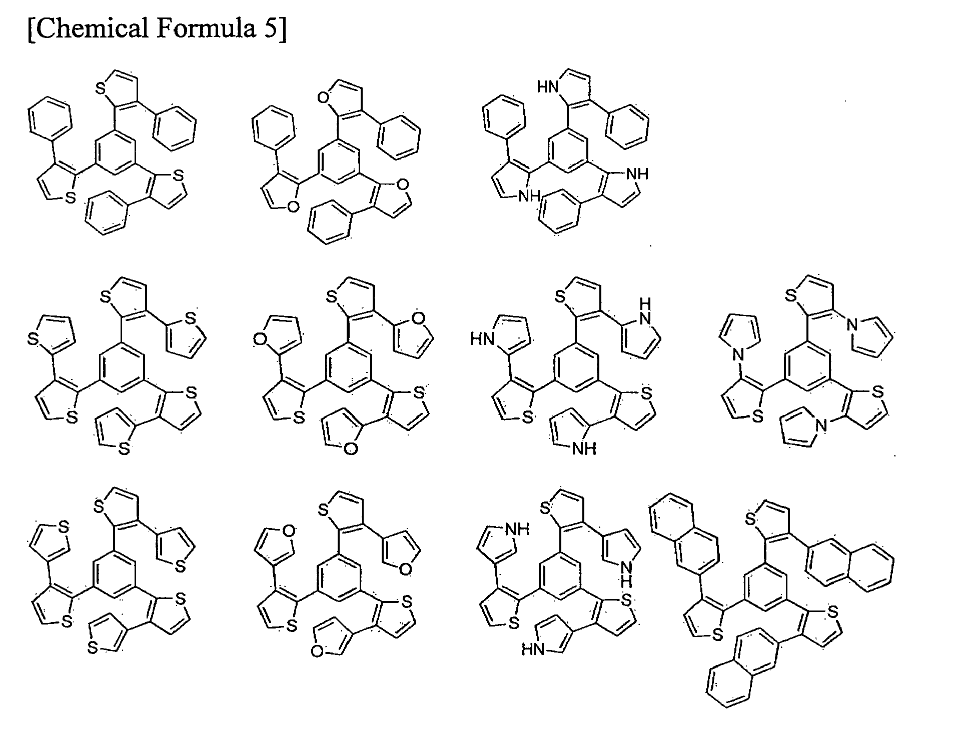

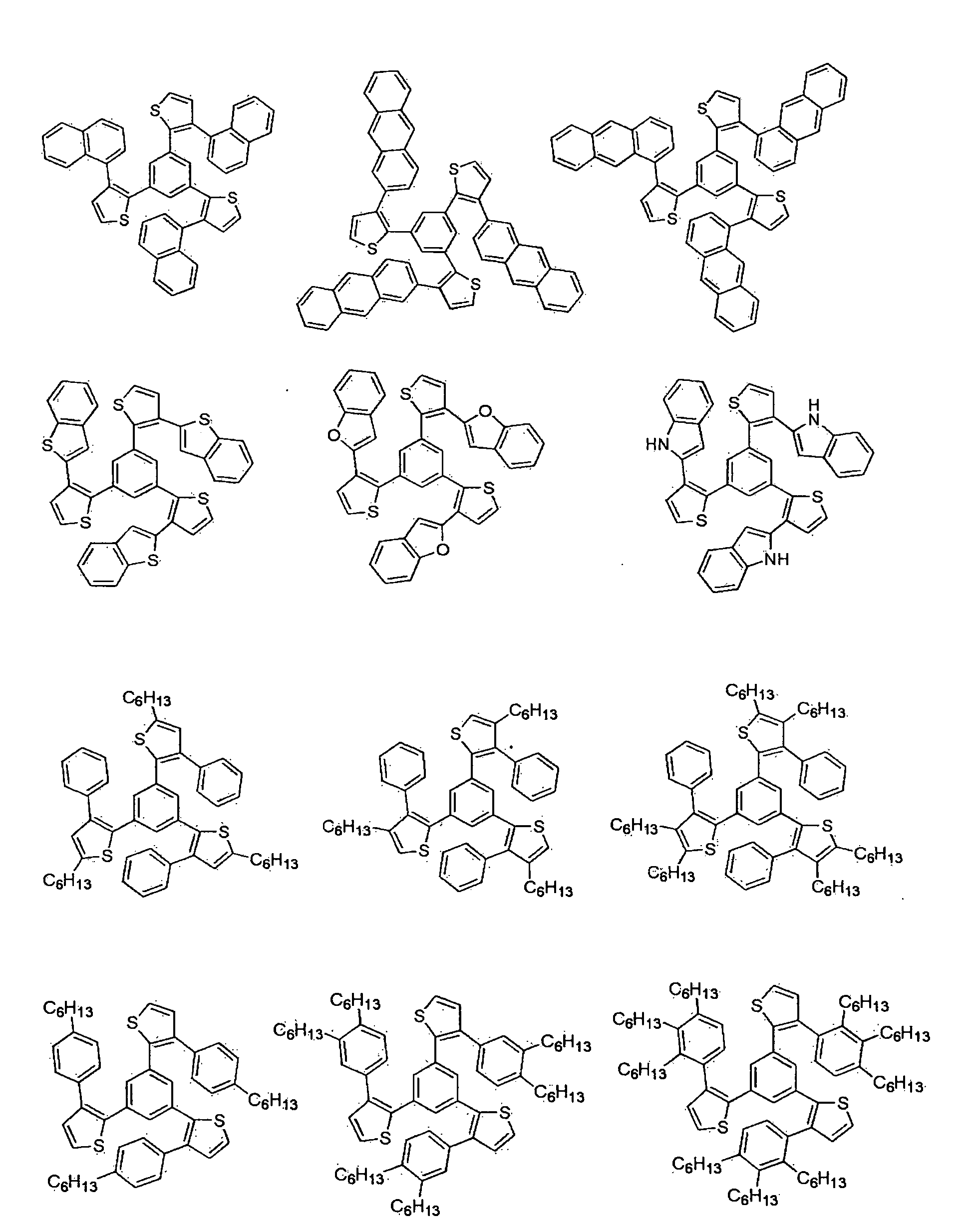

The following are examples of compounds represented by the above formula (2-2).

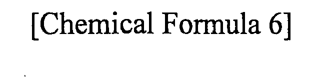

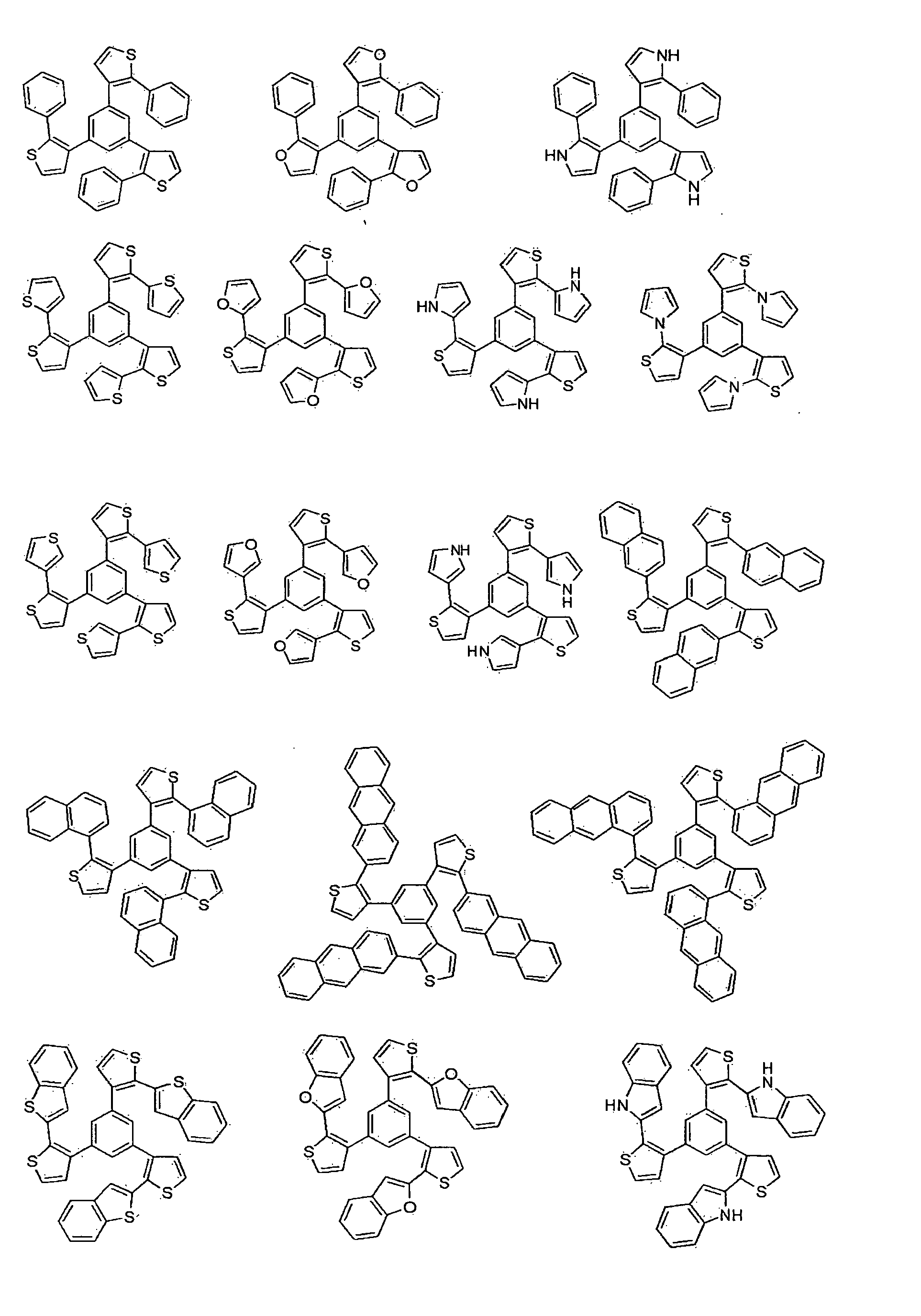

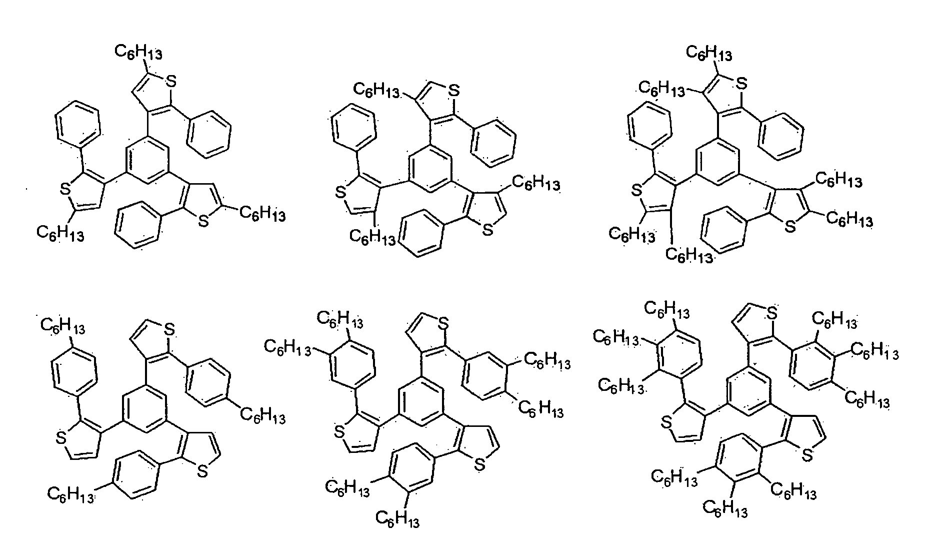

The following are examples of compounds represented by the above formula (2-3).

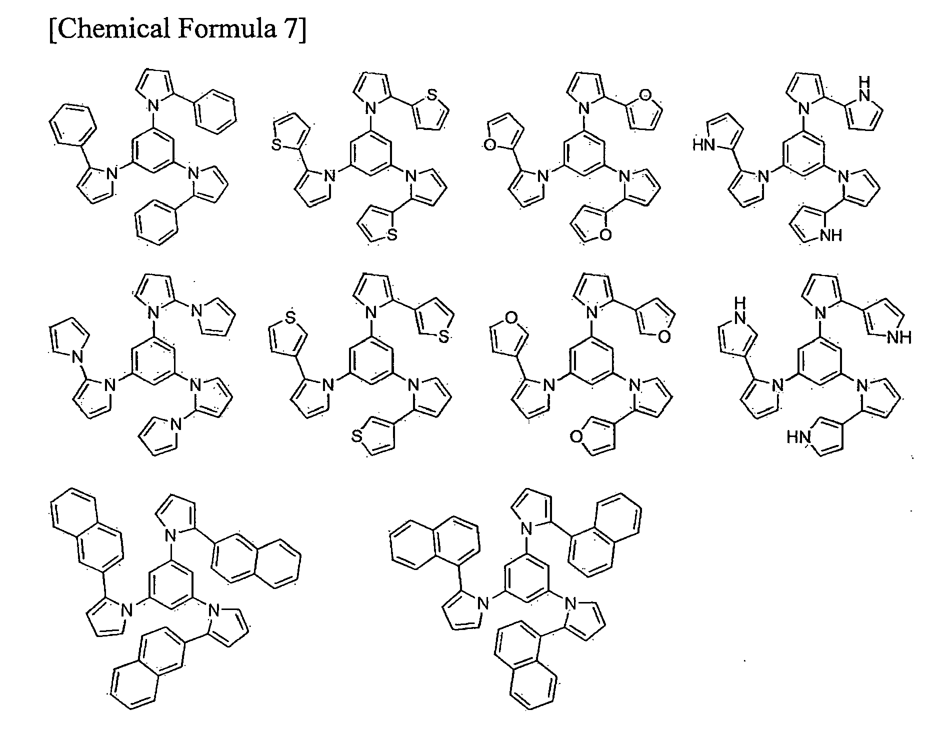

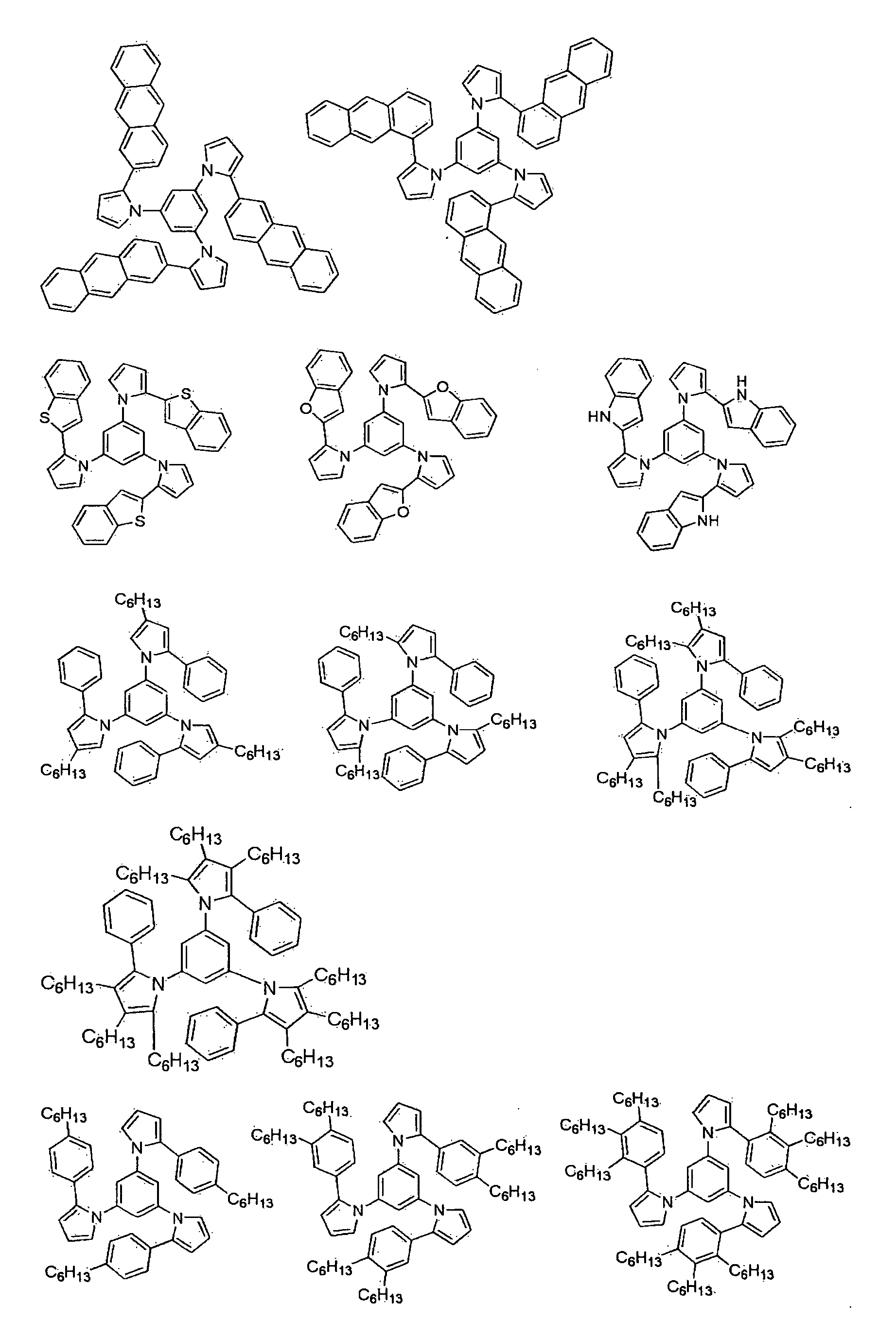

The following are examples of compounds represented by the above formula (2-4).

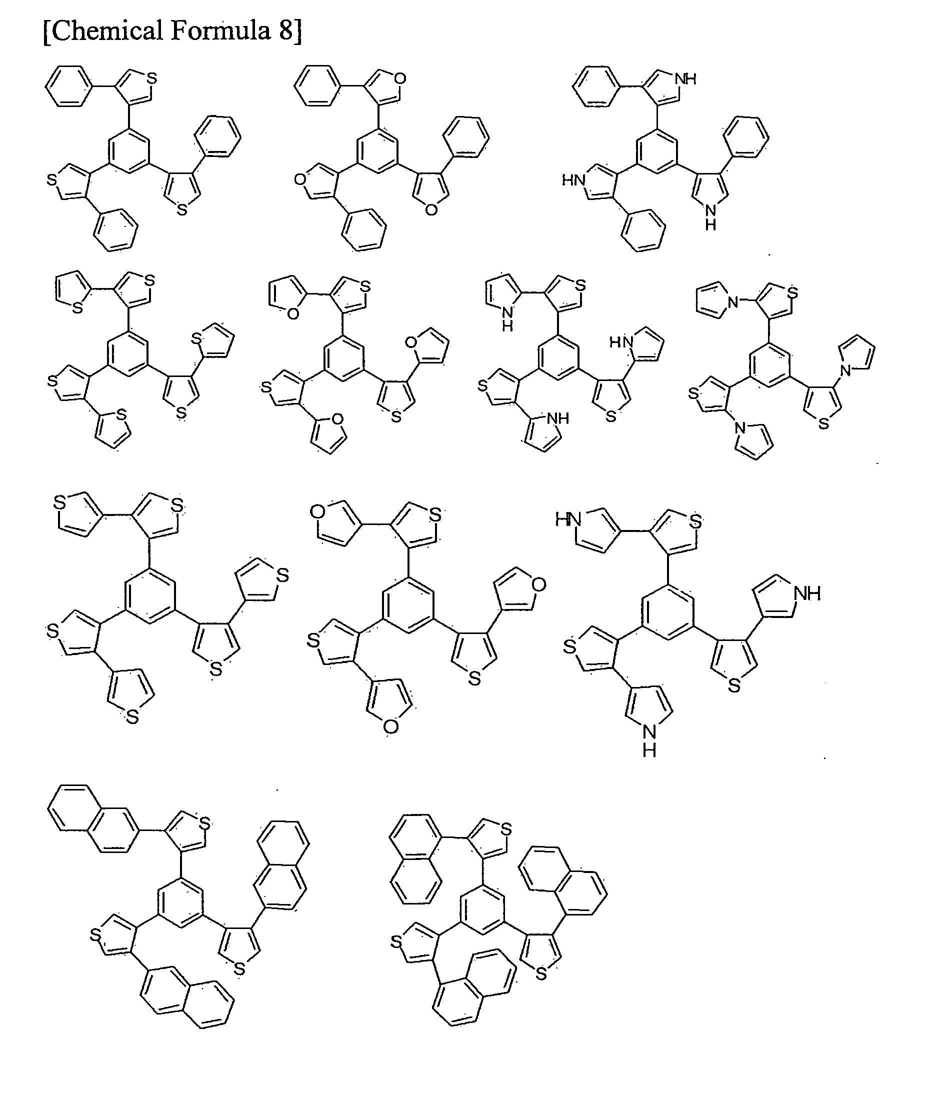

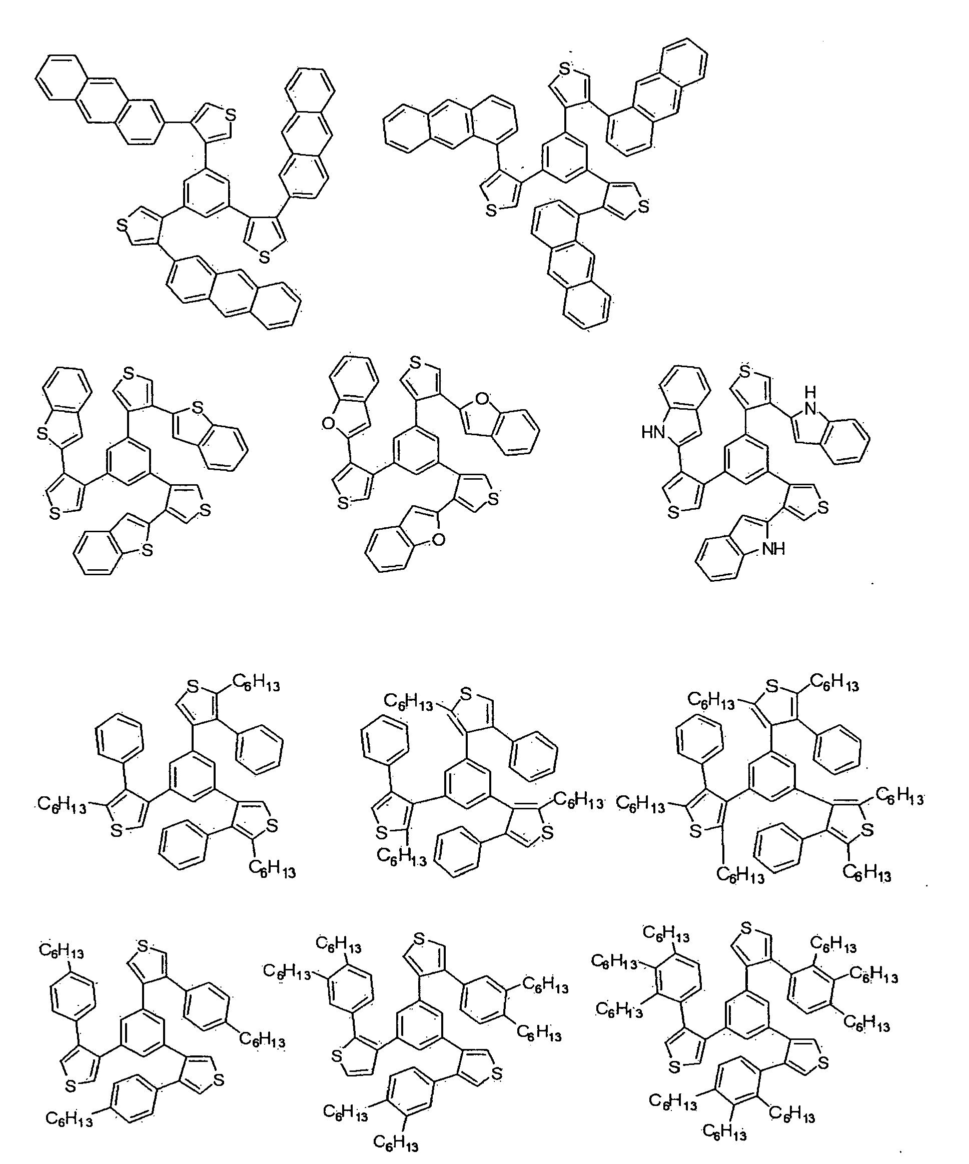

### <Production method>

The compounds of the invention may be produced by any method, and for example, the compounds represented by the above formulas (3-1) to (3-3) can be easily produced by a method comprising cyclization reaction in the presence of an acid or a base. In the above formulas (3-1) to (3-3), Ar^{1*}, Ar^{2*} and Ar^{3*} are, specifically, the same as explained for Ar¹, Ar² and Ar³ above.

Examples of acids include an acetic acid, a hydrochloric acid, a sulfuric acid, a nitric acid, a methanesulfonic acid, a para-toluenesulfonic acid, a trichloroacetic acid, a trifluoroacetic acid, a thionyl chloride, a chlorosilane, a dichlorosilane, a trichlorosilane and a tetrachlorosilane.

Examples of bases include a sodium carbonate, a potassium carbonate, a sodium hydroxide, a potassium hydroxide, a cesium fluoride, a lithium aluminum hydride, a calcium hydride, a sodium hydride and a lithium diisopropylamide.

Preferred among these acids and bases, from the viewpoint of reactivity, are a chlorosilane, a dichlorosilane, a trichlorosilane and a tetrachlorosilane, with a tetrachlorosilane being more preferred.

The amount of acid or base used will usually be 100-5000 mol%, preferably 150-2000 mol% and more preferably 200-1000 mol%, with respect to the total amount of compounds represented by the above formulas (3-1) to (3-3).

The cyclization reaction is preferably carried out in the presence of a solvent. The solvent may be inert to the cyclization reaction, and examples thereof include toluene, xylene, mesitylene, tetrahydrofuran, DMF (dimethylformamide), dioxane, methanol, ethanol, 1-propanol, isopropanol, butanol, acetonitrile and NMP (1-methyl-2-pyrrolidone), with methanol, ethanol, 1-propanol, isopropanol being preferred and ethanol being more preferred, from the viewpoint of improving the yield.

The amount of solvent used will usually be 1-100 fold, and is preferably 2-20 fold, with respect to the total amount of compounds represented by the above formulas (3-1) to (3-3).

The container used for the cyclization reaction may be dried or not dried, but it is preferably heat-dried just before start of the reaction. More preferably, the solvent is added after adding the starting materials and subsequent nitrogen exchange.

The acid or base may be added directly, or dropwise, into the container in which the compounds represented by any of the above formulas (3-1) to (3-3) and the solvent have been mixed, or it may be dissolved in the solvent, but it is preferably added dropwise from the viewpoint of improving the yield.

The reaction temperature for the cyclization reaction is below the boiling point of the solvent, and it is preferably -100 to 50°C, more preferably -20 to 30°C, and most preferably -10 to 20°C.

Upon completion of the cyclization reaction, for example, the obtained reaction mixture may be placed in water, an organic solvent such as toluene, ethyl acetate, diethyl ether or dichloromethane used for extraction, and the obtained organic layer concentrated to obtain the target compound represented by the above formula (1). If necessary, it may be purified by column chromatography, recrystallization, distillation or the like.

When the compound represented by the above formula (2-1), (2-2), (2-3), (2-4), as the compound of the invention, is oxidized with an oxidizing agent or the like, it is possible to obtain a compound represented by the following formula (4-1), (4-2), (4-3), (4-4), (4-5), (4-6), (4-7), having more excellent charge injection and transport properties.

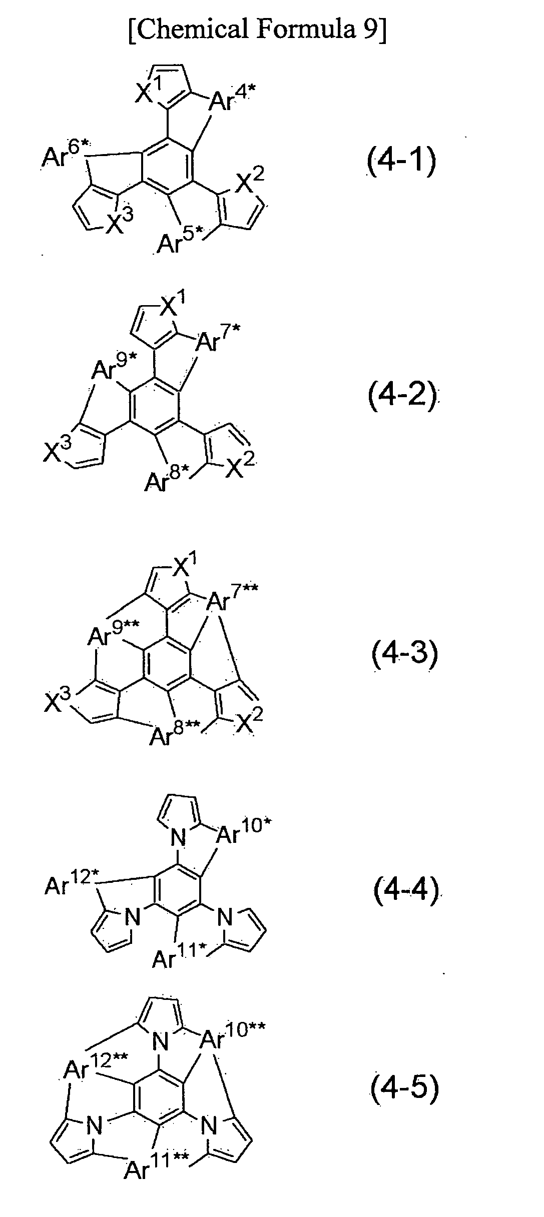

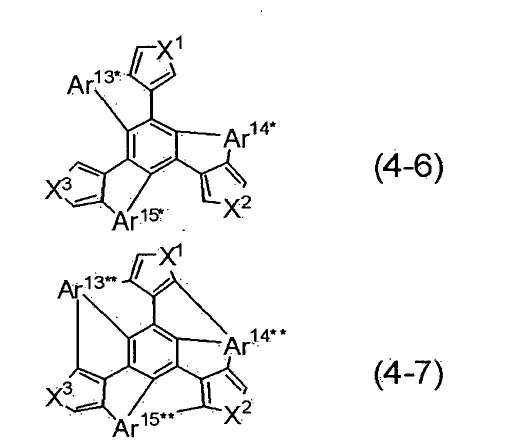

In the formulas, X¹-X³ have the same meanings as explained above. Ar^{4*}, Ar^{5*}, Ar^{6*}, Ar^{7*}, Ar^{8*}, A^{r9*}, Ar^{10*}, Ar^{11*}, Ar^{12*}, Ar^{13*}, Ar^{14*} and Ar^{15*} each independently represent an arylene group or a divalent heterocyclic group. Ar^{7**}, Ar^{8**}, Ar^{9**}, Ar^{10**}, Ar^{11**}, Ar^{12**}, Ar^{13**}, Ar^{14**} and Ar^{15**} each independently represent a trivalent aromatic hydrocarbon group or a trivalent heterocyclic group.

In the above formula (4-1), (4-2), (4-4), (4-6), the arylene groups and the divalent heterocyclic groups represented by Ar^{4*}-Ar^{15*} are specifically, the examples of aryl groups and monovalent heterocyclic groups mentioned for Ar⁴-Ar¹⁵ above, with one hydrogen atom removed.

In the above formula (4-3), (4-5), (4-7), the trivalent aromatic hydrocarbon groups and the trivalent heterocyclic groups represented by Ar^{7**}-Ar^{15**} are specifically, the examples of aryl groups and monovalent heterocyclic groups mentioned for Ar⁴-Ar¹⁵ above, with two hydrogen atoms removed.

As oxidizing agents there may be mentioned halides of transition metals, such as FeCl₃, FeBr₃, V(O)F₃, CuCl₂, PdCl₂ and MnCl₃, as well as their acetic acid salts, nitric acid salts and sulfuric acid salts; halides of typical metals, such as AlCl₃, AlBr₃, MgCl₂, MgBr₂, ZnCl₂, TeCl₄, BBr₃, BCl₃ and BF₃, as well as their acetic acid salts, nitric acid salts and sulfuric acid salts; and catalysts with palladium, platinum or the like supported on carbon.

The following are examples of compounds represented by the above formula (4-1).

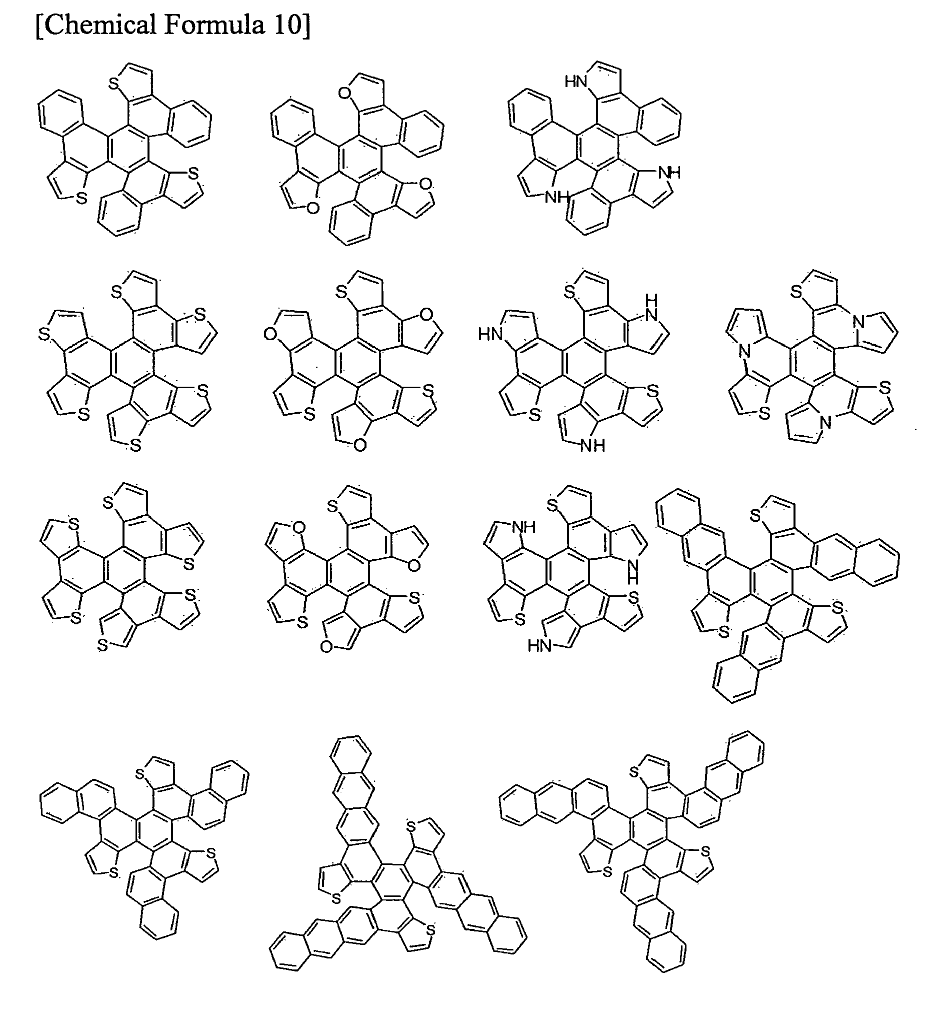

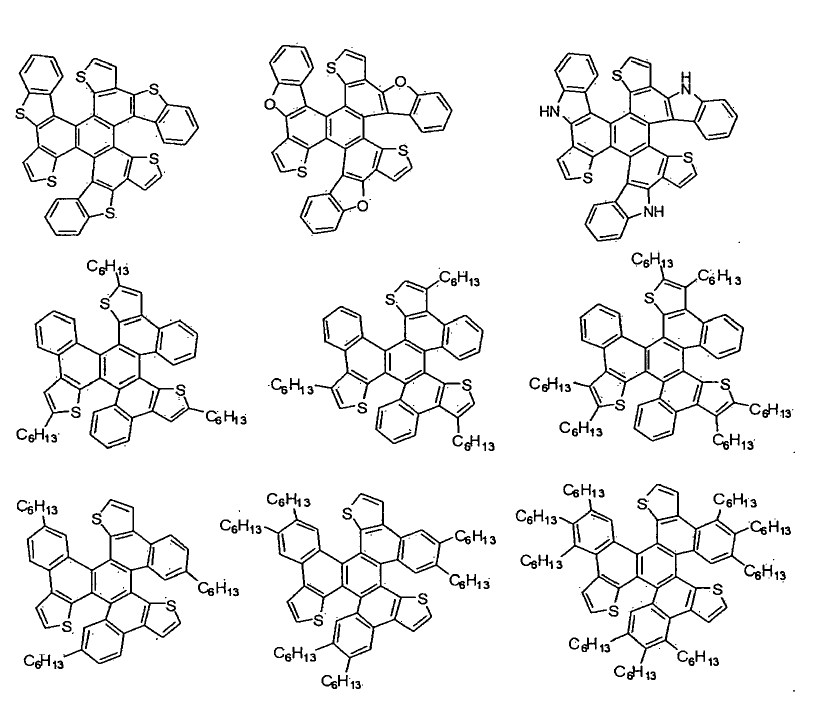

The following are examples of compounds represented by the above formula (4-2).

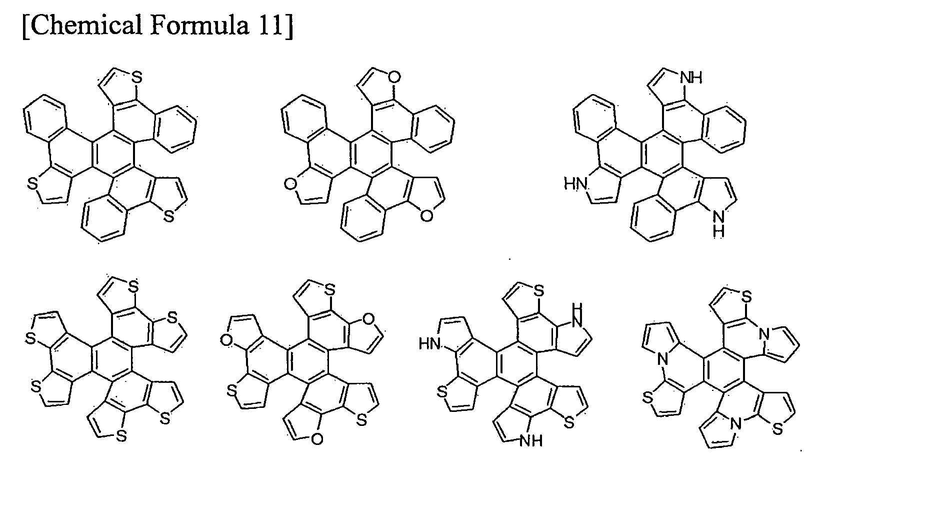

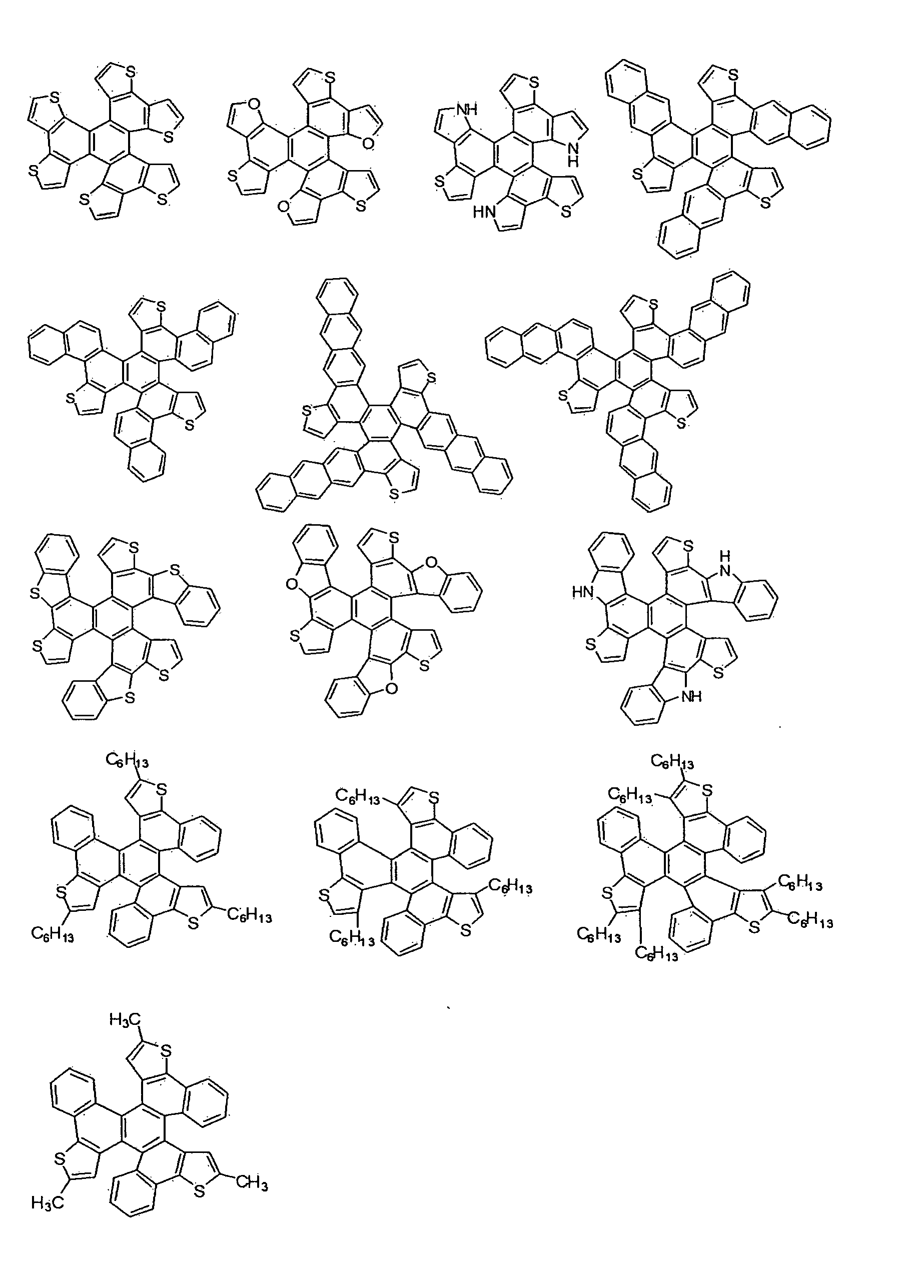

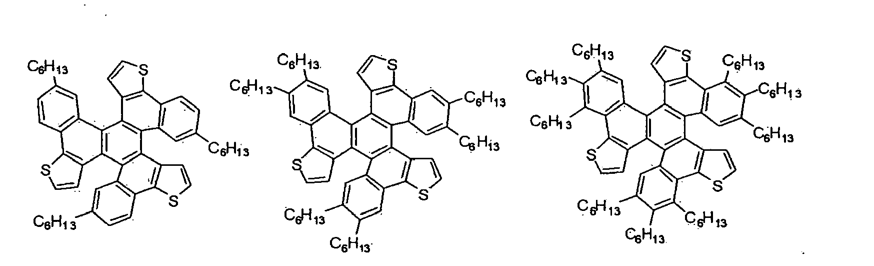

The following are examples of compounds represented by the above formula (4-3).

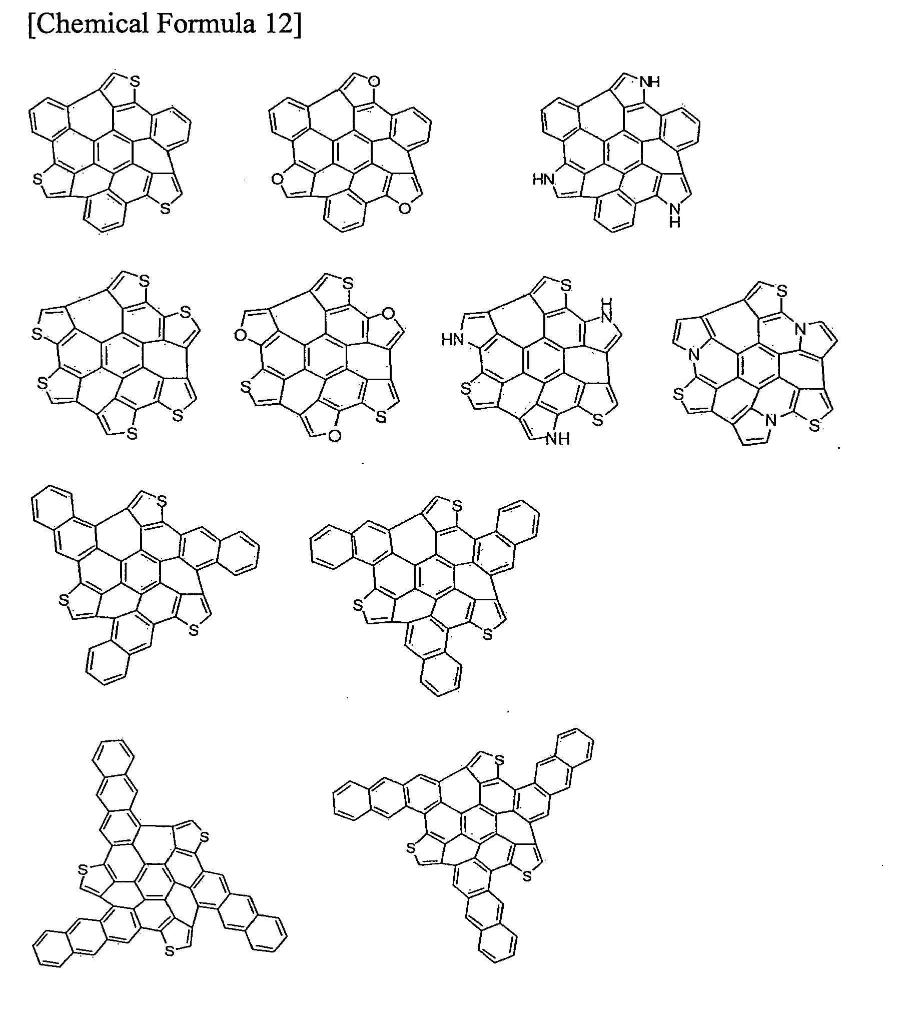

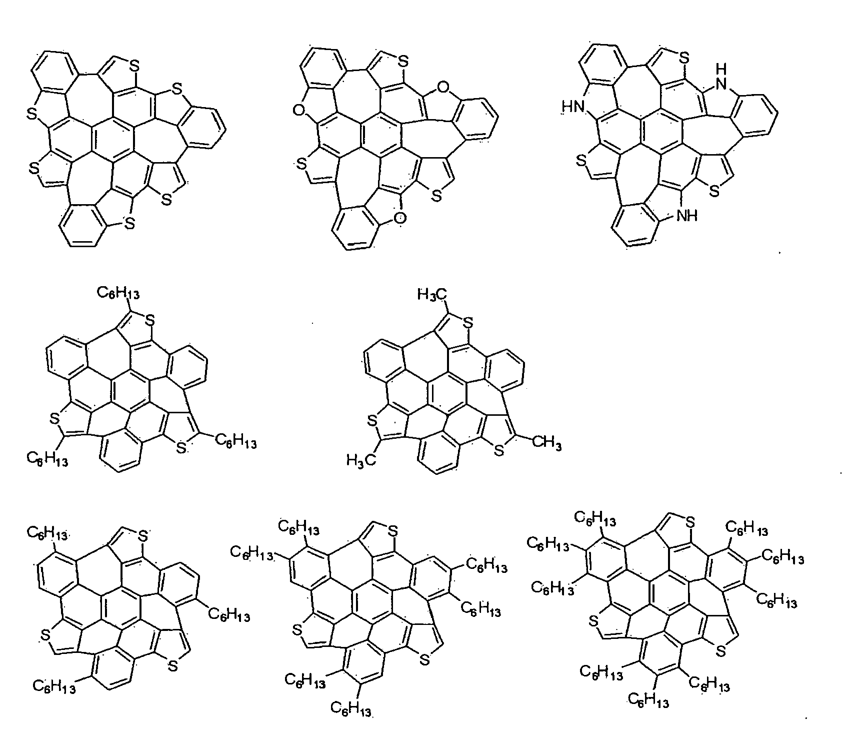

The following are examples of compounds represented by the above formula (4-4).

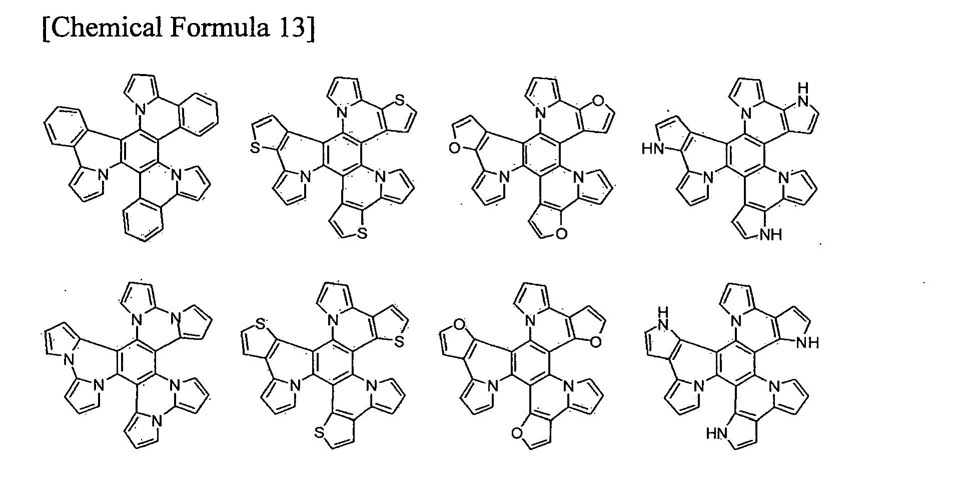

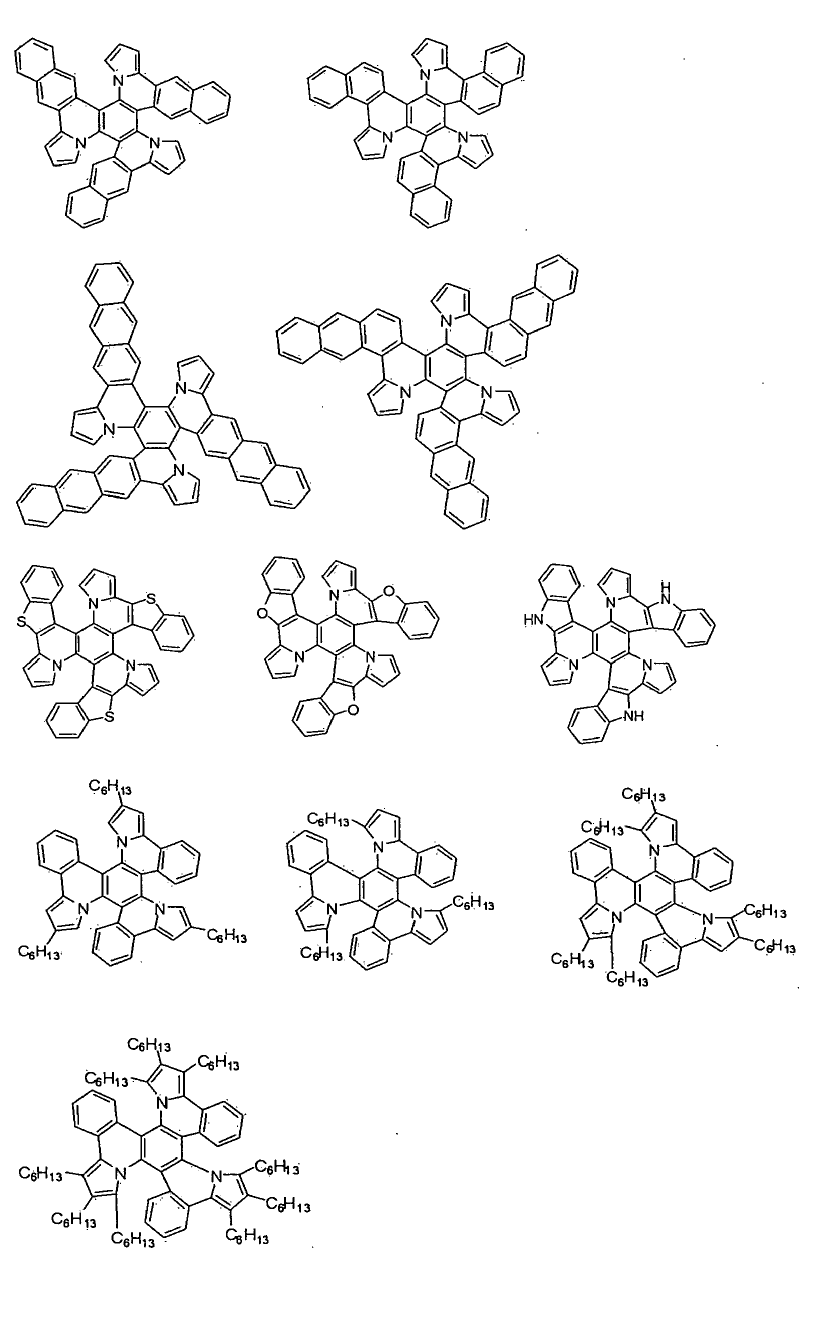

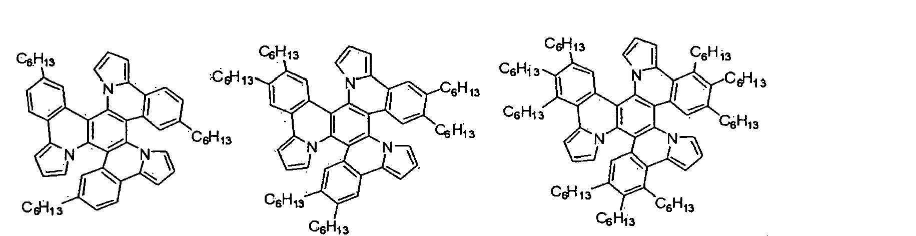

The following are examples of compounds represented by the above formula (4-5).

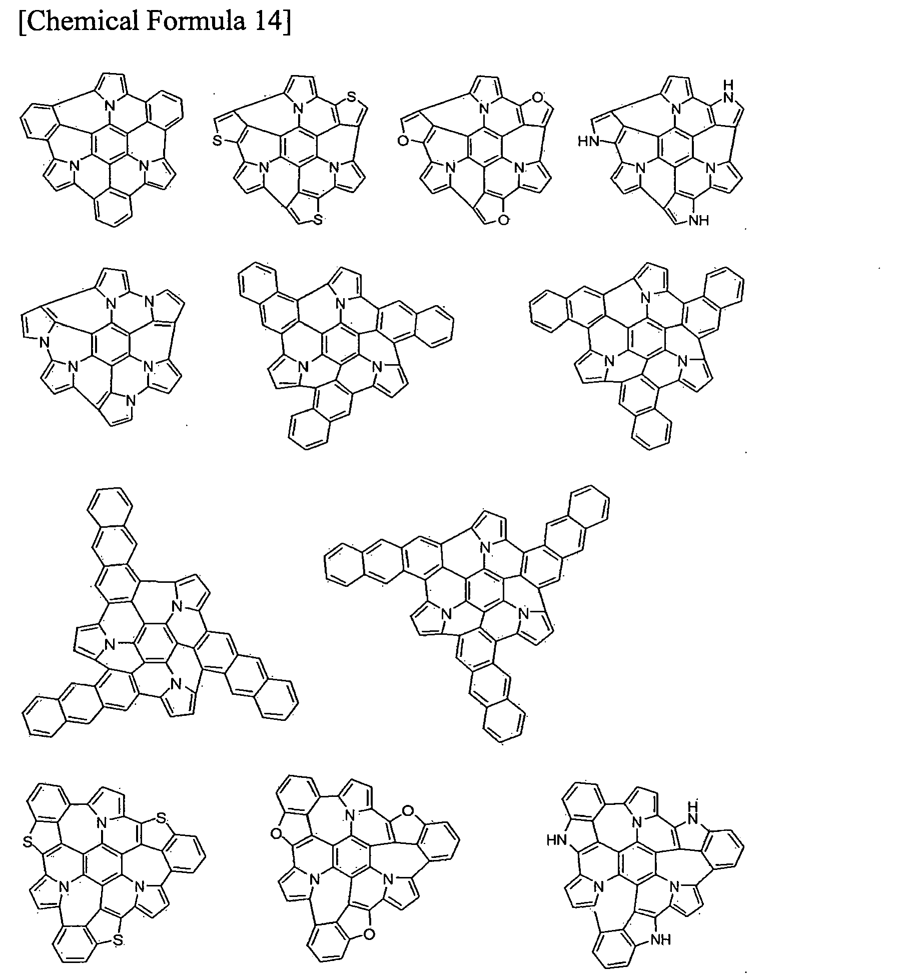

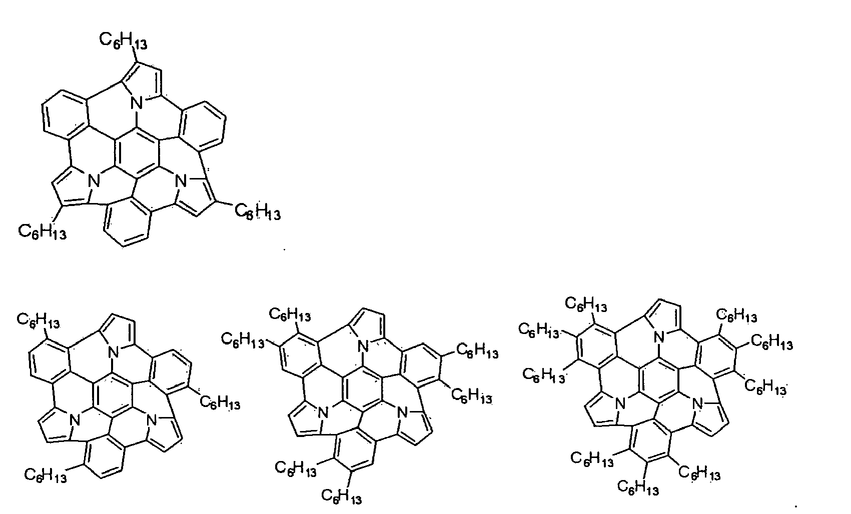

The following are examples of compounds represented by the above formula (4-6).

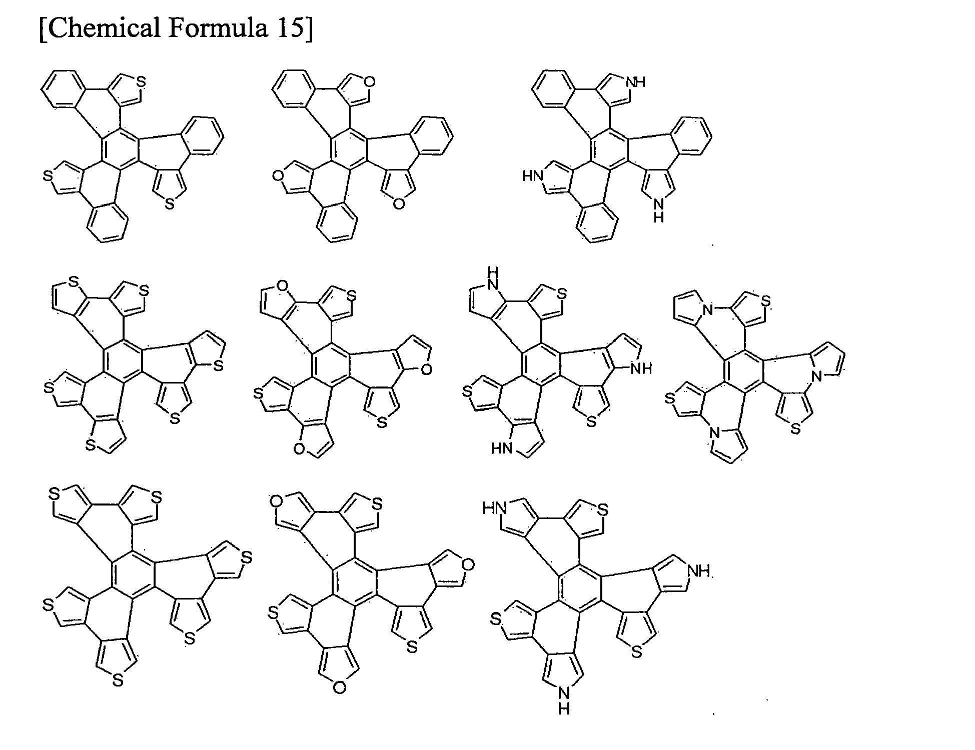

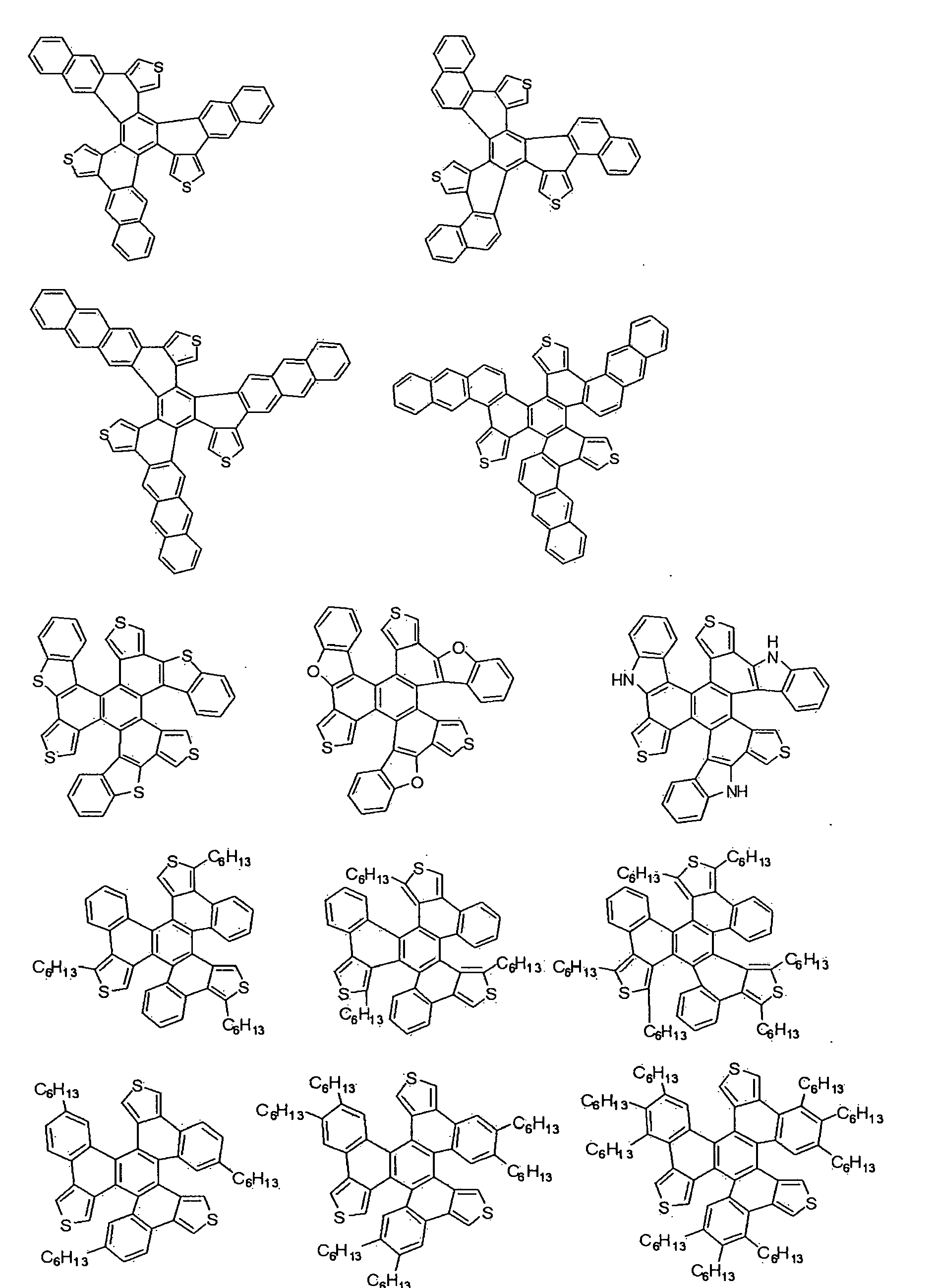

The following are examples of compounds represented by the above formula (4-7).

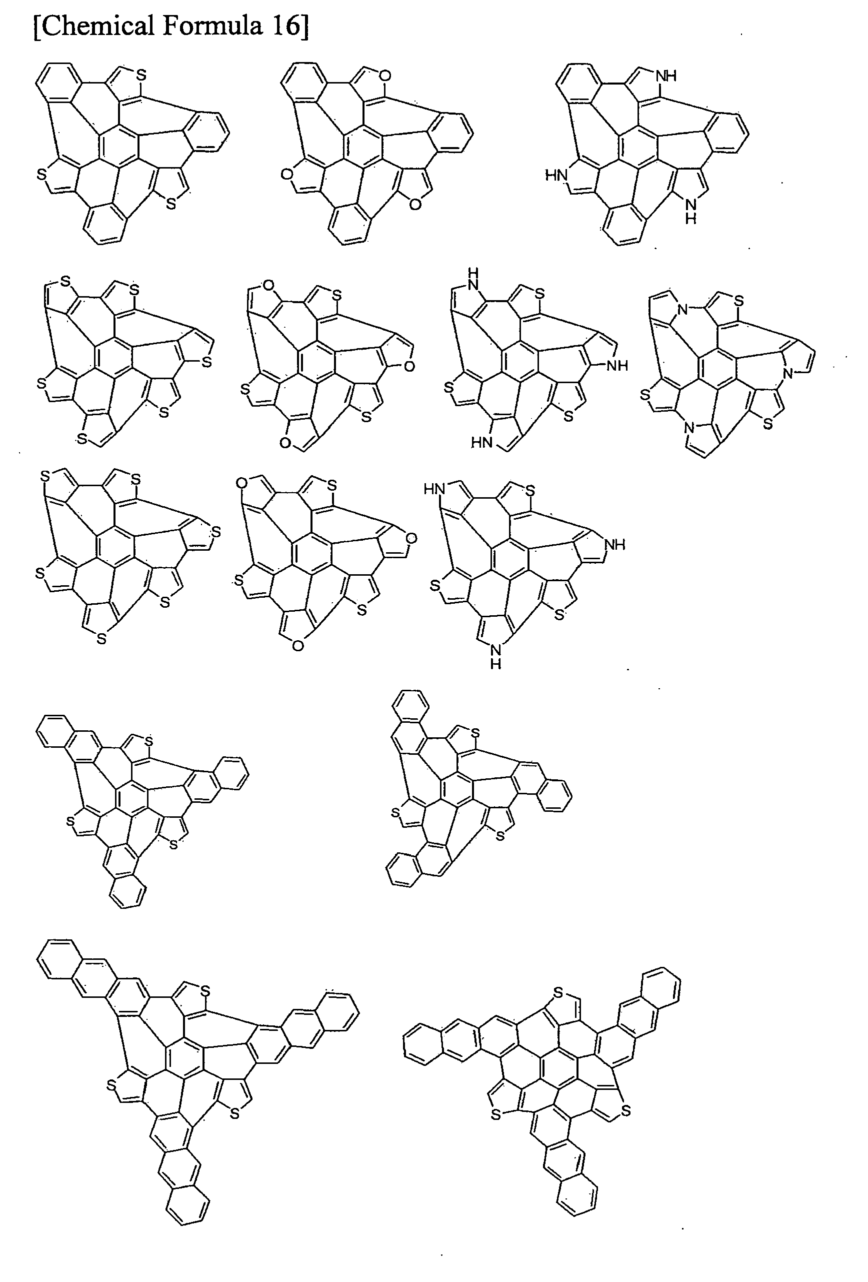

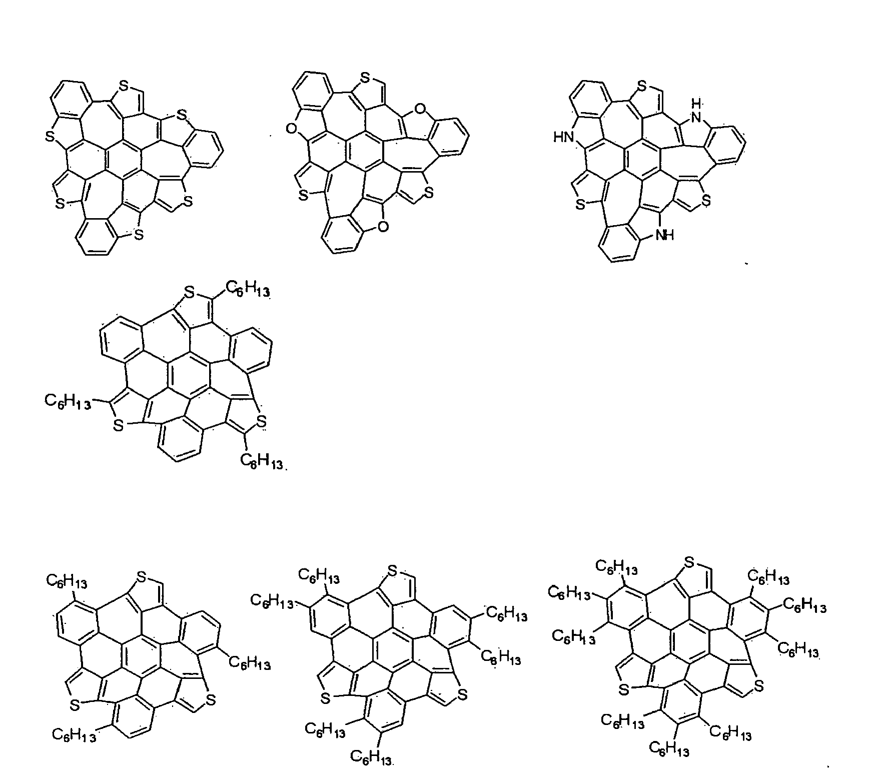

Preferred among these are compounds represented by the above formula (4-1), (4-2), (4-3), and from the viewpoint of charge injection properties, X¹, X², X³ in the compounds represented by the above formulas (4-1), (4-2), (4-3) are more preferably all -S-, -S(=O)- or -S(=O)₂-, and most preferably -S-. In consideration of stability of the molecular structure, Ar⁹-Ar¹² are preferably all phenyl groups.

### <Use>

The compounds of the invention are useful as materials for agricultural chemicals, medicines and other industrial products and as organic semiconductor materials, and they are particularly useful as materials for organic transistors, organic electric field light emitting elements, organic thin-film solar cells, sensors and the like.

### Examples

The present invention will now be further explained by examples, with the understanding that the examples are not limitative on the invention in any way.

### <Example 1>

### (Synthesis of 1,3,5-tris[3'-(2'-phenyl)-thiophen]ylbenzene)

A 5 mL two-necked flask, a Teflon^{R}-coated magnetic stirrer and a blowing tube were placed in a dry oven and heated. After thorough heating, the magnetic stirrer was placed in the flask that had been removed from the dry oven, and the blowing tube was attached. The blowing tube was connected to a pressure reduction/nitrogen line and the entire reactor was exchanged with nitrogen. After allowing the reactor to cool to room temperature, 3-acetyl-2-phenylthiophene (0.50 mmol, 101.6 mg) was placed therein, nitrogen exchange was performed, and then ethanol (1.25 mL) was added. The reactor was cooled to 0°C, and tetrachlorosilane (0.29 mL, 2.5 mmol) was added dropwise. Upon completion of the dropwise addition, the reactor was returned to room temperature and reaction was conducted for 7 hours. Water was added to the reactor to halt the reaction. The obtained aqueous layer was extracted with dichloromethane, and then the obtained organic layer was rinsed with brine.

Magnesium sulfate was added to the obtained organic layer, which was then thoroughly dried. The obtained product was isolated by silica gel column chromatography (eluent: hexane:ethyl acetate = 40:1), to obtain 1,3,5-tris[3'-(2'-phenyl)-thiophen]ylbenzene represented by the following formula: 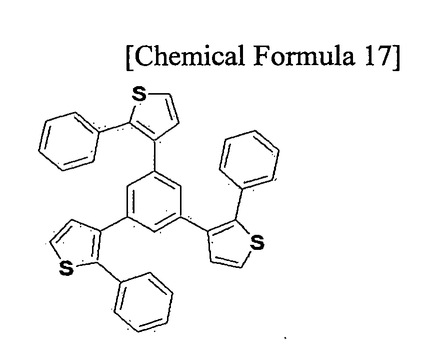 at a yield of 67%.

¹H NMR (CDCl₃)δ6.742 (d, J = 5.1 Hz, 3H, ArH), 7.048 (s, 3H, ArH), 7.206 (d, J = 5.1, 3H, ArH), 7.26- 7.29 (m, 15H, ArH)
¹³C NMR (CDCl₃)δ123.848, 127.328, 128.266, 128.521, 129.229, 130.076, 134.116, 136.675, 137.637, 138.682

IR (KBr) 3102 m, 3055 m, 1594 m, 1534 w, 1491 m, 1444 m, 1354 w, 1073 w, 1031 w, 948 w, 906 w, 877 m, 841 m, 761 s, 728 m, 712 m, 694 s, 665 m, 655 m, 615 w, 584 w, 542 w cm⁻¹

### <Example 2>

### (Synthesis of 1,3,5-tris[2'-(5'-hexyl-3'-phenyl)-thiophen]ylbenzene)

A 5 mL two-necked flask, a Teflon^{R}-coated magnetic stirrer and a blowing tube were placed in a dry oven and heated. After thorough heating, the magnetic stirrer was placed in the flask that had been removed from the dry oven, and the blowing tube was attached. The blowing tube was connected to a pressure reduction/nitrogen line and the entire reactor was exchanged with nitrogen. After allowing the reactor to cool to room temperature, 2-acetyl-3-phenyl-5-hexylthiophene (0.60 mmol, 171.4 mg) was placed therein, nitrogen exchange was performed, and then ethanol (1.0 mL) was added. The reactor was cooled to 0°C, and tetrachlorosilane (0.37 mL, 3.2 mmol) was added dropwise. Upon completion of the dropwise addition, the reactor was returned to room temperature and reaction was conducted for 72 hours. Water was added to the reactor to halt the reaction. The obtained aqueous layer was extracted with dichloromethane, and then the obtained organic layer was rinsed with brine. Magnesium sulfate was added to the obtained organic layer, which was then thoroughly dried. The obtained product was isolated by silica gel column chromatography (eluent: hexane), to obtain 1,3,5-tris[2'-(5'-hexyl-3'-phenyl)-thiophen]ylbenzene represented by the following formula: 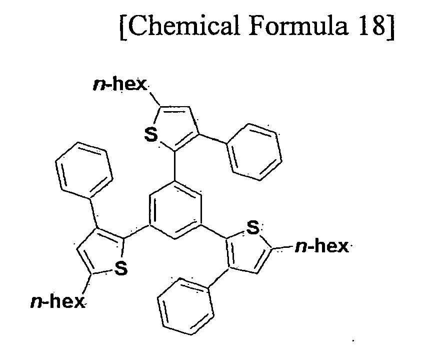 (wherein n-hex represents an n-hexyl group) at a yield of 4%.

¹H NMR (CDCl₃)δ0.896 (t, J = 6.8 Hz, 9H, CH₃), 1.25-1.41 (m, 18H, CH₂CH₂CH₂CH₃), 1.67- 1.74 (m, 6H, CH₂CH2Ar), 2.836 (t, J = 7.5 Hz, 6H, CH₂Ar), 7.072 (s, 3H, ArH), 7.218 (s, 3H, ArH), 7.35- 7.39 (m, 3H, ArH), 7.24- 7.27 (m, 6H, ArH), 7.563 (d, J = 7.3 Hz, 6H, ArH)

¹³C NMR (CDCl₃)δ14.067, 22.566, 28.796, 30.218, 31.574, 31.607, 117.700, 123.355, 126.232, 126.873, 128.689, 136.201, 141.766, 146.722

### <Evaluation>

When organic transistors were fabricated with the 1,3,5-tris[3'-(2'-phenyl)-thiophen]ylbenzene obtained in Example 1 and the 1,3,5-tris[2'-(5'-hexyl-3'-phenyl)-thiophen]ylbenzene obtained in Example 2, and the properties were measured, high charge injection and transport properties were confirmed.

### Industrial Applicability

The compounds of the invention have excellent charge injection and transport properties. In addition, the compounds of the invention generally have excellent stability in air, light stability, acid resistance and heat resistance. According to the production method of the invention it is possible to easily produce the compounds described above.

## Claims

1. A compound represented by the following formula (1): 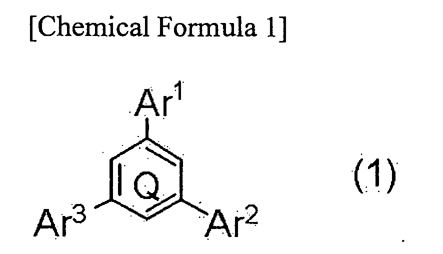 wherein Ar¹, Ar² and Ar³ each independently represent an aryl group or a monovalent heterocyclic group; the aryl group or the monovalent heterocyclic group represented by Ar¹ has an aryl group, a monovalent alicyclic hydrocarbon group or a monovalent heterocyclic group at one position adjacent to the atom that forms Ar¹ and is bonded to a carbon atom forming the benzene ring Q; the aryl group or the monovalent heterocyclic group represented by Ar² has an aryl group, a monovalent alicyclic hydrocarbon group or a monovalent heterocyclic group at one position adjacent to the atom that forms Ar² and is bonded to a carbon atom forming the benzene ring Q; the aryl group or the monovalent heterocyclic group represented by Ar³ has an aryl group, a monovalent alicyclic hydrocarbon group or a monovalent heterocyclic group at one position adjacent to the atom that forms Ar³ and is bonded to a carbon atom forming the benzene ring Q.

2. The compound according to claim 1, wherein Ar¹, Ar² and Ar³ are monovalent heterocyclic groups.

3. The compound according to claim 2, wherein the heterocyclic rings forming the monovalent heterocyclic groups represented by Ar¹, Ar² and Ar³ are 5-membered rings.

4. The compound according to claim 3, wherein the heterocyclic rings forming the monovalent heterocyclic groups represented by Ar¹, Ar² and Ar³ are each independently a thiophene ring, a furan ring, a pyrrole ring, a thiazole ring or an oxazole ring.

5. The compound according to claim 4 which is represented by any one of the following formulas (2-1) to (2-4): 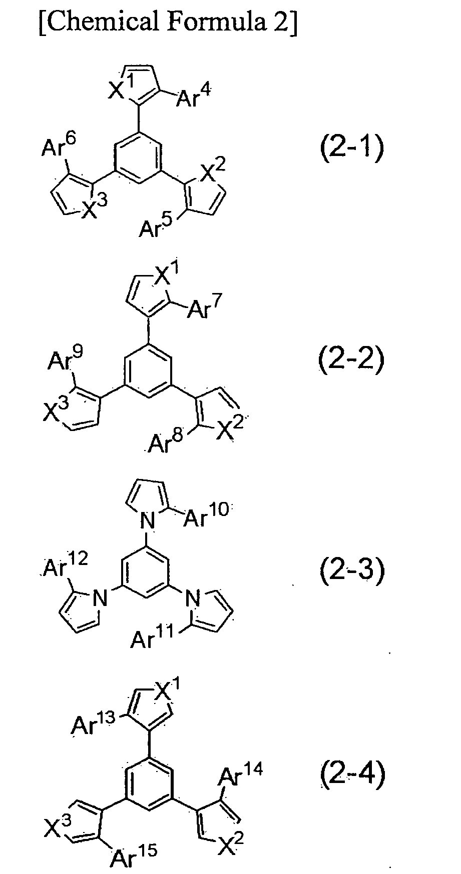 wherein X¹, X² and X³ each independently represent -S(=O)ₙ-, -O- or -N(R²)-; n represents an integer of 0-2; R² represents a hydrogen atom, an alkyl group, an alkoxy group, an alkylthio group, an alkylamino group, an aryl group, an aryloxy group, an arylalkyl group, an arylalkoxy group, an arylamino group, or a monovalent heterocyclic group; Ar⁴, Ar⁵, Ar⁶, Ar⁷, Ar⁸, Ar⁹, Ar¹⁰, Ar¹¹, Ar¹², Ar¹³, Ar¹⁴ and Ar¹⁵ each independently represent an aryl group or a monovalent heterocyclic group.

6. The compound according to claim 5, wherein X¹, X² and X³ are -S-, -S(=O)- or -S(=O)₂-.

7. A method for producing the compound according to any one of claims 1 to 6, which comprises making a compound represented by any of the following formulas (3-1) to (3-3) undergo a cyclization reaction in the presence of an acid or a base: 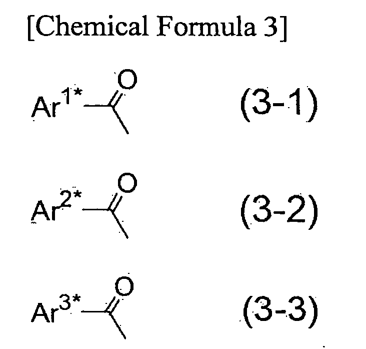 wherein Ar^{1*}, Ar^{2*} and Ar^{3*} each independently represent an aryl group or a monovalent heterocyclic group; the aryl group or the monovalent heterocyclic group represented by Ar^{1*} has an aryl group, a monovalent alicyclic hydrocarbon group or a monovalent heterocyclic group at one position adjacent to the atom that forms Ar^{1*} and is bonded to the acetyl group; the aryl group or the monovalent heterocyclic group represented by Ar^{2*} has an aryl group, a monovalent alicyclic hydrocarbon group or a monovalent heterocyclic group at one position adjacent to the atom that forms Ar^{2*} and is bonded to the acetyl group; the aryl or the monovalent heterocyclic group represented by Ar^{3*} has an aryl group, a monovalent alicyclic hydrocarbon group or a monovalent heterocyclic group at one position adjacent to the atom that forms Ar^{3*} and is bonded to the acetyl group.
